# NEW EUROPEAN PATENT SPECIFICATION

(11) **EP 2 520 177 B2**
(45) Date of publication and mention of the opposition decision: **26.02.2020**
(45) Mention of the grant of the patent: 31.05.2017
(21) Application number: 10821437.0
(22) Date of filing: 05.11.2010
(51) Int. Cl.: A23L 19/00, A23L 33/12, A61K 31/202, A23P 10/30, A23L 33/00

(54) **NUTRITIONAL COMPOSITIONS COMPRISING FRUIT FLAKES CONTAINING DOCOSAHEXAENOIC ACID**
ERNÄHRUNGSZUSAMMENSETZUNGEN MIT OBSTFLOCKEN MIT DOCOSAHEXAENSÄURE
COMPOSITIONS NUTRITIONNELLES COMPRENANT DES FLOCONS DE FRUITS CONTENANT DE L'ACIDE DOCOSAHEXÉNOÏQUE

(30) Priority: 29.12.2009 US 290609 P
(43) Date of publication of application: 07.11.2012
(73) Proprietor: Société des Produits Nestlé S.A., 1800 Vevey (CH)
(72) Inventor: CHÁVEZ, Gabriela, C. P. 76902 Municipio Corregidora Querétaro (MX)
(74) Representative: Elkington and Fife LLP
(86) International application number: PCT/MX2010/000126
(87) International publication number: WO 2011/081509

(56) References cited:
- WO-A1-2010/011712
- FR-A- 1 324 053
- US-A- 4 587 132
- US-A1- 2003 207 004
- US-A1- 2004 091 599
- US-A1- 2010 021 607
- RUALES J ET AL: "EVALUATION OF THE NUTRITIONAL QUALITY OF FLAKES MADE OF BANANA PULP AND FULL-FAT SOYA FLOUR", FOOD CHEMISTRY, ELSEVIER LTD, NL, vol. 31, no. 1, 1 January 1990 (1990-01-01), pages 31-43, XP008009175, ISSN: 0308-8146, DOI: DOI:10.1016/0308-8146(90)90005-O
- DATABASE GNPD [Online] Mintel; April 2009 (2009-04), Anonymous: "Baby rice with banana", XP002637926, Database accession no. 1081688
- DATABASE GNPD [Online] Mintel; October 2004 (2004-10), Anonymous: "Reisbrei Apfel-Birne Apple & Pear Rice Pudding", XP002637927, Database accession no. 310115
- DATABASE GNPD [Online] Mintel; March 2009 (2009-03), Anonymous: "Omega supplement", XP002637928, Database accession no. 1051325

## Description

### BACKGROUND

The present disclosure relates in general to health and nutrition. More specifically, the present disclosure relates to fruit flakes that consist of fruit and encapsulated docosahexaenoic acid derived from fish oil, and methods for producing and using said fruit flakes.

There are many types of nutritional compositions currently on the market. Nutritional compositions can be directed towards particular types of consumers, for example the young, the elderly, the athletic, etc., based on specific ingredients of the nutritional composition. The nutritional compositions contain flakes of dehydrated fruits, which are known in the market. However, there is still a need for fruit flakes that contain additional micronutrients and macronutrients.

Ruales et al. (Food Chemistry, Elsevier Ltd, NL, vol. 31, no. 1, 1 January 1990, pages 31-42) relates to evaluation of the nutritional quality of flakes made of banana pulp and full-fat soya flour.

### BRIEF DESCRIPTION OF THE INVENTION

Fruit flakes and methods for producing and using said fruit flakes are provided. In a general embodiment, the present disclosure provides a fruit flake characterized in that it consists of fruit and encapsulated docosahexaenoic acid derived from fish oil. The docosahexaenoic acid is incorporated directly in the fruit flakes. The docosahexaenoic acid is derived from fish oil and is encapsulated. The fruit flakes can be made from any suitable fruit including apple, mango, peach, etc.

The fruit flake is free from preservatives. Even without preservatives, the fruit flakes can be stable (for example, do not deteriorate or oxidize) for 12 months or more.

In one embodiment, the present disclosure provides a fruit flake characterised in that it consists of fruit and encapsulated docosahexaenoic acid derived from fish oil. The fruit flake can be apple flake, mango flakes, peach flakes or a combination thereof. The fruit flake is free from preservatives.

The fruit flakes are 100% fruit with encapsulated docosahexaenoic acid derived from fish oil and can be supplied in a convenient packet. The fruit flakes can be poured into a vessel and water and can be added to produce a fruit purée. The fruit purée can have a suitable consistency and flavor for infants and contribute to the development of children's eyes and brain. Moreover, addition of water to create a fruit purée provides a carer a feeling of compromise for making foods for the child.

In yet another embodiment, the present disclosure provides a method for producing fruit flakes which consists of fruit and encapsulated docosahexaenoic acid derived from fish oil. The method comprises puréeing a fruit to form a fruit purée, adding docosahexaenoic acid to the fruit purée, drying the fruit purée, and cutting the dried fruit purée into fruit flakes.

In one embodiment, the method further comprises sifting the fruit flakes. The method can further comprise packing the fruit flakes in any suitable container. The method can be carried out in aseptic conditions.

In another embodiment, the present disclosure provides a method for producing a food for infants. The method comprises providing fruit flakes that consist of fruit and encapsulated docosahexaenoic acid derived from fish oil and adding a liquid to the fruit flakes to form a fruit purée.

In an alternative embodiment, the present disclosure provides a method for providing nutrition for an infant. The method comprises providing fruit flakes that consist of fruit and encapsulated docosahexaenoic acid derived from fish oil, adding a liquid to the fruit flakes to form a fruit purée, and administering the fruit purée to the infant.

One advantage of the present disclosure is that it provides an improved fruit flake that includes encapsulated docosahexaenoic acid derived from fish oil.

Another advantage of the present disclosure is that it provides a method for producing an improved fruit flake.

Yet another advantage of the present disclosure is that it provides an improved method for improving the nutrition of an infant.

The further features and advantages are described herein, and will be clear from the following detailed description and the drawings.

### BRIEF DESCRIPTION OF THE DRAWINGS

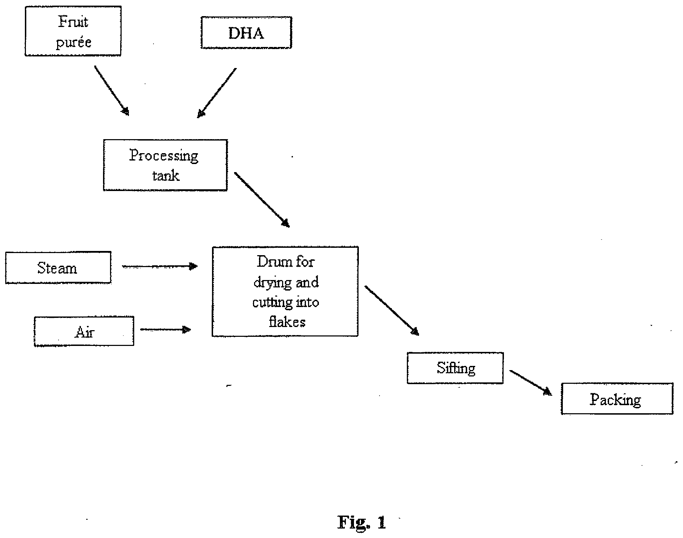

Fig. 1 shows a scheme of one embodiment of a manufacturing process for making fruit flakes.

### DETAILED DESCRIPTION

The present disclosure relates to fruit flakes and methods for making fruit flakes. In a general embodiment, the present disclosure provides a fruit flake characterised in that it consists of fruit and encapsulated docosahexaenoic acid ("DHA") derived from fish oil. The fruit flakes can be made from any suitable fruit including apple, mango, peach, etc.

The DHA can be incorporated directly in the fruit flakes by mixing or combining during the fruit flake manufacturing process. The DHA is derived from fish oil. The DHA can be, for example, in the form of encapsulated DHA powder (for example, tuna oil microencapsulated with gelatin).

Docosahexaenoic acid (commonly known as DHA; 22:6(ω-3), all-ciss-docosa-4,7,10,13,16,19-hexaenoic acid; trivial name cervonic acid) is an omega-3 essential fatty acid. With respect to its chemical structure, DHA is carboxylic acid with a chain of 22 - carbons and six cis double bonds. The first double bond is positioned on the third carbon from the omega end.

DHA is the most abundant essential fatty acid (polyunsaturated fatty acids ("PUFAs")) in the brain and the retina. It comprises 40% of PUFAs in the brain and 60% of PUFAs in the retina. 50% of the weight of the plasma membrane of the neuron is composed of DHA. DHA contributes to development of the brain and of the eyes especially in children under 2 years of age.

Fish oils are rich in DHA. Most of the DHA in fish and more complex organisms derived from photosynthetic microalgae and heterotrophs, and becomes more and more concentrated in the organisms as they move up to the food chain. DHA is also manufactured commercially from microalgae. The manufactured DHA that uses microalgae is vegetarian. Most animals make very little DHA via the metabolism, including humans. However, small amounts are manufactured internally through the consumption of α-linolenic acid, an omega-3 fatty acid found in plants, animals and milk.

As used herein, the term "fruit flake" includes fruit particles. The fruit particles can have any suitable diameter, density and moisture content. The fruit particles can be partially or completely soluble in a liquid. Preferably, the fruit particles are dehydrated.

The fruit flake can be made from any suitable fruit such as apples, mangos, peaches, etc. The fruit flake is free from preservatives yet is stable for 12 months or more.

The fruit flakes are 100% fruit with encapsulated DHA derived from fish oil and supplied in a convenient packet. The fruit flakes can be poured into a vessel and water can be added to the fruit flakes in any suitable amount to produce a fruit purée. The fruit purée can have suitable consistency and flavor for infants and can contribute to the development of the brain and eyes of the infant.

The present invention offers the advantage of providing a stable means for supply of DHA by incorporating DHA directly in a fruit flake. Incorporation can be effected using any suitable mixing process. Surprisingly, it was found that incorporation of DHA directly in the fruit flake provides better stability for DHA and prevents oxidation of DHA, which easily suffers mechanical damage that causes the gelatin capsule to break, thus exposing the oil to the air and turning the DHA rancid.

As shown in Fig. 1, one method for producing fruit flakes comprises puréeing a fruit to form a fruit purée, adding DHA to the fruit purée (for example, mixing or combining in a processing tank), drying the fruit purée, and cutting the dry fruit purée into fruit flakes. The dry fruit purée can be made into fruit flakes using any suitable method such as grinding or cutting the dry fruit purée. The methods can be carried out in aseptic conditions. The dry fruit flakes must combine critical parameters of thickness, density and moisture to maintain stability during their useful life.

For example, the fruit purée can be put in one or more drying drums and a flow of air provided for each of the drying drums. The air flow, the quality and the model can be modified as necessary for drying the fruit purée to the desired physical composition.

The revolutions per minute (rpm) of the drying drums can also be controlled for affecting the conditions of drying of the fruit purée. For example, the rpm can provide good results.

Once the fruit purée has been dried and has been transformed into flakes by cutting, grinding, etc., the method can further comprise sifting the fruit flakes. This can be used for obtaining fruit flakes that have a specific size or diameter. The fruit flakes can be packed in any suitable container.

In another embodiment, the present disclosure provides a method for producing an infant food. The method comprises providing fruit flakes that consist of fruit and encapsulated docosahexaenoic acid derived from fish oil and adding a liquid to the fruit flakes to form a fruit purée. For example, 16 grams of fruit flakes can be added to a bag of the feed bag type for a single portion of portions. The portion can contain 18 mg of DHA and can provide, when mixed with 80 ml of water, a fruit purée ready for eating and specially designed for infants or young children.

In an alternative embodiment, the present disclosure provides a method for providing nutrition to an infant. The method comprises providing fruit flakes that consist of fruit and encapsulated docosahexaenoic acid derived from fish oil, adding a liquid to the fruit flakes to form a fruit purée, and administering the purée to the infant. The liquid can include water, milk, a formula or any suitable fruit juice. Any suitable amount of liquid can be combined or mixed with the fruit flakes to form a fruit purée that has a desired consistency. The fruit purée can contribute to the development of the brain and eyes of the infant.

It must be understood that various changes and modifications to the preferred embodiments described herein will be apparent to a person skilled in the art. Said changes and modifications can be made without decreasing its intended advantages.

## Claims

1. A fruit flake **characterized in that** it consists of fruit and encapsulated docosahexaenoic acid derived from fish oil.

2. The fruit flake as claimed in claim 1, further **characterized in that** the fruit flake is selected from the group consisting of apple flakes, mango flakes, peach flakes and a combination thereof.

3. The fruit flake as claimed in claim 1, further **characterized in that** it is stable for 12 months or more.

4. A method for producing fruit flakes which consist of fruit and encapsulated docosahexaenoic acid derived from fish oil, said method comprising:
puréeing a fruit to form a fruit purée;
adding docosahexaenoic acid to the fruit purée;
drying the fruit purée; and
cutting the dry fruit purée into fruit flakes.

5. The method as claimed in claim 4, **characterized in that** it further comprises sifting the fruit flakes.

6. The method as claimed in claim 4, further **characterized in that** it comprises packing the fruit flakes.

7. The method as claimed in claim 4, further **characterized in that** the fruit is selected from the group consisting of apple, mango, peach and a combination thereof.

8. The method as claimed in claim 4, further **characterized in that** the method is carried out in aseptic conditions.

9. Fruit flakes obtained by the method as claimed in any of claims 4 to 8.

10. A method for producing an infant food, said method comprising:
providing fruit flakes including docosahexaenoic acid as claimed in any of claims 1 to 3 or 9; and
adding a liquid to the fruit flakes to form a fruit purée.

11. A method for supplying nutrition to an infant, said method comprising:
providing fruit flakes including docosahexaenoic acid as claimed in any of claims 1 to 3 or 9;
adding a liquid to the fruit flakes to form a fruit purée; and
administering the fruit purée to an infant.

## Patentansprüche

1. Fruchtflocke, **dadurch gekennzeichnet, dass** sie Früchte und von Fischöl abgeleitete verkapselte Docosahexaensäure umfasst.

2. Fruchtflocke nach Anspruch 1, ferner **dadurch gekennzeichnet, dass** die Fruchtflocke ausgewählt ist aus der Gruppe bestehend aus Apfelflocken, Mangoflocken, Pfirsichflocken und einer Kombination davon.

3. Fruchtflocke nach Anspruch 1, ferner **dadurch gekennzeichnet, dass** sie 12 Monate oder länger stabil ist.

4. Verfahren zur Herstellung von Fruchtflocken, die aus Frucht und von Fischöl abgeleiteter gekapselter Docosahexaensäure bestehen, wobei das Verfahren umfasst:
Pürieren einer Frucht zur Zubereitung eines Fruchtpürees;
Zugeben von Docosahexaensäure zum Fruchtpüree;
Trocken des Fruchtpürees; und
Schneiden des trockenen Fruchtpürees zu Fruchtflocken.

5. Verfahren nach Anspruch 4, **dadurch gekennzeichnet, dass** es ferner das Sieben der Fruchtflocken umfasst.

6. Verfahren nach Anspruch 4, ferner **dadurch gekennzeichnet, dass** es das Verpacken der Fruchtflocken umfasst.

7. Verfahren nach Anspruch 4, ferner **dadurch gekennzeichnet, dass** die Frucht ausgewählt ist aus der Gruppe bestehend aus Apfel, Mango, Pfirsich und einer Kombination davon.

8. Verfahren nach Anspruch 4, ferner **dadurch gekennzeichnet, dass** das Verfahren unter aseptischen Bedingungen ausgeführt wird.

9. Fruchtflocken, gewonnen anhand des Verfahrens nach einem der Ansprüche 4 bis 8.

10. Verfahren zur Herstellung einer Babynahrung, wobei das Verfahren umfasst:
Bereitstellen von Fruchtflocken, die Docosahexaensäure nach einem der Ansprüche 1 bis 3 oder 9 enthalten; und
Zugeben einer Flüssigkeit zu den Fruchtflocken zur Bildung eines Fruchtpürees.

11. Verfahren zum Verabreichen von Nahrung an ein Baby, wobei das Verfahren umfasst:
Bereitstellen von Fruchtflocken, die Docosahexaensäure nach einem der Ansprüche 1 bis 3 oder 9 enthalten;
Zugeben einer Flüssigkeit zu den Fruchtflocken zur Bildung eines Fruchtpürees; und
Verabreichen des Fruchtpürees an ein Baby.

## Revendications

1. Flocon de fruit **caractérisé en ce qu'**il est constitué de fruit et d'acide docosahexaénoïque encapsulé dérivé d'huile de poisson.

2. Flocon de fruit selon la revendication 1, **caractérisé en outre en ce que** le flocon de fruit est choisi dans le groupe constitué de flocons de pomme, flocons de mangue, flocons de pêche et une combinaison de ceux-ci.

3. Flocon de fruit selon la revendication 1, **caractérisé** en outre **en ce qu**'il est stable pendant 12 mois ou plus.

4. Procédé de production de flocons de fruit qui sont constitués de fruit et d'acide docosahexaénoïque encapsulé dérivé d'huile de poisson, ledit procédé comprenant :
la réduction en purée d'un fruit pour former une purée de fruit ;
l'ajout d'acide docosahexaénoïque à la purée de fruit ;
le séchage de la purée de fruit ; et
la découpe de la purée de fruit sèche en flocons de fruit.

5. Procédé selon la revendication 4, **caractérisé en ce qu**'il comprend en outre le tamisage des flocons de fruit.

6. Procédé selon la revendication 4, **caractérisé** en outre **en ce qu**'il comprend le conditionnement des flocons de fruit.

7. Procédé selon la revendication 4, **caractérisé en outre en ce que** le fruit est choisi dans le groupe constitué de pomme, mangue, pêche et une combinaison de celles-ci.

8. Procédé selon la revendication 4, **caractérisé en outre en ce que** le procédé est réalisé dans des conditions aseptiques.

9. Flocons de fruit obtenus par le procédé selon l'une quelconque des revendications 4 à 8.

10. Procédé de production d'un aliment pour nourrissons, ledit procédé comprenant :
la fourniture de flocons de fruit incluant de l'acide docosahexaénoïque selon l'une quelconque des revendications 1 à 3 ou 9 ; et
l'ajout d'un liquide aux flocons de fruit afin de former une purée de fruit.

11. Procédé de fourniture d'une nutrition à un nourrisson, ledit procédé comprenant:
la fourniture de flocons de fruit incluant de l'acide docosahexaénoïque selon l'une quelconque des revendications 1 à 3 ou 9 ;
l'ajout d'un liquide aux flocons de fruit afin de former une purée de fruit ; et
l'administration de la purée de fruit à un nourrisson.
